Europäisches Patentamt

European Patent Office

Office européen des brevets

(11) Veröffentlichungsnummer: **0 095 124**
**A1**

(12) **EUROPÄISCHE PATENTANMELDUNG**

(21) Anmeldenummer: **83104834.3**

(22) Anmeldetag: **17.05.83**

(51) Int. Cl.³: **G 01 N 24/08**
**A 61 B 5/05**

(30) Priorität: **26.05.82 DE 3219832**

(43) Veröffentlichungstag der Anmeldung:
**30.11.83 Patentblatt 83/48**

(84) Benannte Vertragsstaaten:
**AT BE CH DE FR GB IT LI LU NL SE**

(71) Anmelder: **Bruker Medizintechnik GmbH**
**Silberstreifen**
**D-7512 Rheinstetten-Forchheim(DE)**

(72) Erfinder: **Knüttel, Bertold**
**Baumgartenstrasse 5**
**D-7512 Rheinstetten 2(DE)**

(74) Vertreter: **Patentanwälte Kohler - Schwindling - Späth**
**Hohentwielstrasse 41**
**D-7000 Stuttgart 1(DE)**

(54) Verfahren zur nicht-invasiven Ermittlung von Messwerten innerhalb eines lebenden Körpers.

(57) Das Verfahren dient zur nicht-invasiven Ermittlung chemischer und/oder physikalischer Zustände innerhalb des gesamten, unversehrten, lebenden, tierischen oder menschlichen Körpers unter Verwendung magnetischer Resonanz. Dabei werden hochauflösende Messungen an frei wählbaren, an beliebigen Orten innerhalb des lebenden Körpers liegenden Meßpunkten durch Anwendung einer lokalen magnetischen Resonanz vorgenommen. Insbesondere können auf diese Weise die Temperatur und der pH-Wert an beliebigen Meßpunkten innerhalb des zu untersuchenden Körpers bestimmt und überwacht werden.

Anmelder:

Bruker Medizintechnik GmbH
Silberstreifen
7512 Rheinstetten-Forchheim

Stuttgart, 5. Mai 1983

P 4210 W/We

Vertreter:

Kohler-Schwindling-Späth
Patentanwälte
Hohentwielstraße 41
7000 Stuttgart 1

## Verfahren zur nicht-invasiven Ermittlung von Meßwerten innerhalb eines lebenden Körpers

Die Erfindung geht aus von einem Verfahren zur nicht-invasiven Ermittlung chemischer und/oder physikalischer Zustände innerhalb des gesamten, unversehrten, lebenden, tierischen oder menschlichen Körpers unter Verwendung Magnetischer Resonanz mit einem homogenen Konstantmagnetfeld und einem Hochfrequenzmagnetfeld, wobei einzelne Bereiche des Körpers durch eine hochauflösende Magnetische-Resonanz-Messung ausgemessen werden und frei wählbare, an beliebigen Orten innerhalb des lebenden Körpers liegende Meßpunkte durch Anwendung einer auf auswählbare Volumenbereiche begrenzten Magnetischen Resonanz (LMR, Local Magnetic Resonance, Lokalisierte Magnetische Resonanz) untersucht werden.

Es ist bekannt, Gewebeproben im Hinblick auf chemische und/oder physikalische Zustände, beispielsweise den pH-Wert, die chemische Zusammensetzung, die Temperatur u.dgl. zu untersuchen. Dabei unterscheidet man zwischen sog. in-vitro-Messungen und den sog. in-vivo-Messungen. Bei den in-vitro-Messungen wird ein nicht lebendes Gewebe im Reagenzglas untersucht, während die in-vivo-Messung grundsätzlich am lebenden Gewebe, also am oder im lebenden tierischen oder menschlichen Körper erfolgt.

Weiterhin unterscheidet man zwischen invasiven und nicht-invasiven Meßmethoden. Bei den invasiven Meßmethoden wird eine Sonde, ein Katheter o.dgl. in das zu untersuchende Gewebe eingeführt, was bei in-vivo-Messungen mit einer erheblichen Belästigung und u.U. Verletzung des zu untersuchenden Körpers verbunden sein kann. Derartige Messungen scheiden demzufolge insbesondere dann aus, wenn empfindliche Organe betroffen sind oder sind nur unter großen Schwierigkeiten, etwa bei gleichzeitiger Anästhesie o.dgl., durchzuführen.

Zur nicht-invasiven Messung ist es bekannt, den Körper durchdringende Wellen bzw. Strahlen zu verwenden, beispielsweise Ultraschall oder Röntgenstrahlen. Während die ersten jedoch nur relativ ungenaue Informationen über den Innenraum des zu untersuchenden Körpers ergeben, sind die zweitgenannten Methoden mit einer Strahlenbelastung des Körpers verbunden.

Es ist weiter bekannt, zur Untersuchung von Geweben die sog. Magnetische Resonanz zu verwenden, bei der eine zu untersuchende Probe gleichzeitig einem homogenen Konstantmagnetfeld und gleichzeitig einem Hochfrequenzmagnetfeld ausgesetzt wird. Während die Magnetische Resonanz früher überwiegend für in-vitro-Messungen eingesetzt wurde, sind in den letzten Jahren auch in-vivo-Messungen bekannt geworden, die üblicherweise als Kernresonanz-Tomographie (NMR-Tomographie) bezeichnet werden.

Bei der NMR-Tomographie wird dem homogenen Konstantmagnetfeld ein sog. Gradientenfeld überlagert, welches bewirkt, daß die effektive Konstantmagnetfeldstärke in der Achse des Magnetfeldes linear zunimmt. Wird nun der zu untersuchende Körper mit einem Hochfrequenzfeld einer bestimmten Frequenz beaufschlagt, gerät nur die Ebene des zu untersuchenden Körpers in Resonanz, die eine Konstantmagnetfeldstärke aufweist, bei der das eingestrahlte Hochfrequenzmagnetfeld gerade eine Magnetische Resonanz auslöst. Durch Verwendung zusätzlicher Gradientenfelder, die eine Richtung senkrecht zum Konstantmagnetfeld haben, ist es möglich, flächenhafte Abbildungen einer zu untersuchenden Querschnittsebene in mehreren Winkellagen herzustellen, so daß schlußendlich ein Bild konstruiert werden kann, das den aus der Röntgen-Computertomographie entsprechenden Abbildungen entspricht, wobei die Verwendung von Magnetischer Resonanz jedoch den erheblichen Vorteil hat, daß die Strahlenbelastung, die die Röntgenmethode aufweist, entfällt.

Die Tomographie mit Hilfe der Magnetischen Resonanz liefert in der vorstehend beschriebenen Vorgehensweise jedoch nur sehr grobe Informationen, da bei der Erstellung der genannten Querschnittsbilder lediglich die in der Querschnittsebene vorhandene Spindichte aufgezeichnet wird. Es ist daher auch lediglich erforderlich, eine Magnetische Resonanz relativ geringer Auflösung zu verwenden.

4 0095124

Aus der Firmenschrift "Topical Magnetic Resonance Spectroscopy" der Firma Oxford Research Systems ist schließlich
noch ein Verfahren bekannt geworden, mit dem hochauflösende
Kernresonanzmessungen einzelner Bereiche eines lebenden
tierischen Körpers möglich sind. Dazu wird eine Probespule
auf die Oberfläche des tierischen Körpers aufgebracht, so
daß ein im einzelnen nicht definierter Bereich an und in der
Nähe der Oberfläche des tierischen Körpers durch hochauflösende Kernresonanzmessungen ausgemessen werden kann. Das
bekannte Verfahren gestattet jedoch keine Untersuchung
definierter Meßpunkte, insbesondere nicht frei wählbarer
Meßpunkte im Inneren des Meßkörpers.

Der Erfindung liegt demgegenüber die Aufgabe zugrunde, ein
Verfahren anzugeben, bei dem physiologisch-diagnostische
Messungen unmittelbar verwertbarer Parameter an beliebigen
Meßpunkten innerhalb des menschlichen Körpers möglich sind.

Diese Aufgabe wird erfindungsgemäß dadurch gelöst, daß
einmal aus einem Parameter des gemessenen Kernresonanzspektrums die Temperatur innerhalb der ausgewählten Volumenbereiche ermittelt wird und gemäß einer nebengeordneten Lösung
der zugrundeliegenden Aufgabe der pH-Wert innerhalb der
ausgewählten Volumenbereiche festgestellt wird.

Durch das erfindungsgemäße Verfahren ist es damit erstmals
möglich, die außergewöhnlichen analytischen Möglichkeiten
der hochauflösenden Kernresonanzspektroskopie auch bei
nicht-invasiven in-vivo-Messungen an beliebigen Stellen im
Inneren des tierischen oder menschlichen Körpers dafür
einzusetzen, um direkt diagnostisch verwertbare Meßwerte zu
erzielen.

Durch die in den Unteransprüchen angegebene Merkmale sind weitere Vorteile erzielbar.

So werden in bevorzugter Ausgestaltung der Erfindung zur Ausmessung definierter, frei wählbarer Meßpunkte Meßverfahren verwendet, wie sie aus der Literatur für in-vitro-Messungen an sich teilweise bekannt sind und teilweise auf älteren Patentanmeldungen der Anmelderin beruhen. Diesen Verfahren ist gemeinsam, daß die freie Auswählbarkeit des Meßpunktes mit rein elektronischen Mitteln vorgenommen werden kann, so daß auch automatisierte Messungen möglich sind und die Lage des zu untersuchenden Körpers während der Messung nicht verändert zu werden braucht.

In besonders bevorzugter Ausgestaltung des erfindungsgemäßen Verfahrens werden die Temperatur und/oder der pH-Wert am Orte des jeweiligen Meßpunktes bestimmt. Hierdurch ist eine außerordentlich genaue und hochselektive Überwachung dieser physikalischen bzw. chemischen Zustände am lebenden Körper möglich. Wird beispielsweise die Temperatur gemessen, eröffnet dies ganz neue Anwendungsmöglichkeiten bei der Behandlung von schweren Erkrankungen, insbesondere Tumoren mittels Hyperthermie, weil durch das erfindungsgemäße Verfahren überwacht werden kann, ob irgendwelche Bereiche des menschlichen Körpers oder vor unzulässig hohen Temperaturen besonders zu schützende Bereiche, wie beispielsweise das Gehirn, noch eine die Gesundheit nicht beeinträchtigende Temperatur aufweisen. Bei dem weiteren erfindungsgemäßen Verfahren, bei dem der pH-Wert festgestellt wird, ist damit nicht nur die Diagnose von körperlichen Zuständen, beispielsweise definierter Organe oder Organbereiche möglich, das erfindungsgemäße Verfahren kann darüber hinaus insoweit auch zur Überwachung der Wirksamkeit von Behandlungen, insbesondere medikamentösen Behandlungen, eingesetzt werden.

Die genannten Verfahren sind aufgrund ihrer rein elektronischen Vorgehensweise auch außerordentlich schnell, so daß eine Gesamtuntersuchung eines lebenden Körpers mittels der erfindungsgemäßen Verfahren relativ schnell erfolgen kann. Beispielsweise kann die für die spektrometrische Erfassung der Temperatur in einem Volumenbereich des Körpers erforderliche Zeit für ein Volumen, das etwa einen Kubikcentimeter betragen mag, nur etwa einige 10 Sekunden ausmachen.

Da die bekannten Verfahren der Magnetischen Resonanz die Resonanz von verschiedenen Kernarten ausnutzen können, können die erfindungsgemäßen Verfahren mit einer Vielzahl von Kernarten eingesetzt werden, so daß eine entsprechende Vielzahl von entweder körpereigenen oder körperfremden Substanzen verwendet werden kann, sofern diese nur magnetische Resonanzsignale liefern. Zur Erhöhung der Meßgenauigkeit und der Signalausbeute kann dabei auch in vorteilhafter Weise ein "NMR-Kontrastmittel" verabfolgt, beispielsweise injiziert werden, wobei wiederum eine Vielzahl von Kernarten zur Verfügung steht. Unter "Kontrastmittel" wird dabei eine Substanz verstanden, die eine besonders ausgeprägte Abhängigkeit eines Parameters des NMR-Spektrums, beispielsweise der chemischen Verschiebung, von der Temperatur bzw. dem pH-Wert aufweist.

Die Meßgenauigkeit, insbesondere Eichgenauigkeit bzw. die Variationsmöglichkeit der Messungen, beispielsweise für die gleichzeitige Erfassung mehrerer Parameter, kann weiter dadurch erhöht werden, daß erfindungsgemäß mehrere unterschiedliche Kernarten gleichzeitig ausgemessen werden.

Weiter kann in bevorzugter Ausgestaltung der Erfindung nicht
nur der jeweilige physikalische und/oder chemische Zustand
eines einzigen Meßpunktes erfaßt werden, es ist durch einfaches Fortschalten der Meßpunkte vielmehr ebenso möglich,
komplette Flächenraster der ermittelten Meßwerte, beispielsweise von Temperatur oder pH-Wert zu erzeugen, die vorzugsweise einer Querschnittsebene durch den zu untersuchenden
Körper entsprechen. Damit kann dann etwa die Temperaturverteilung in der Ebene oder die Verteilung des pH-Wertes
dargestellt werden.

Schließlich werden in bevorzugter Ausgestaltung der Erfindung noch zusätzliche Homogenisierungsmittel, d.h. sogenannte Shimspulen verwendet, um den oder die einzelnen zu untersuchenden Meßpunkte bezüglich des wirksamen Konstantmagnetfeldes weiter zu homogenisieren, so daß die physikalischen
und/oder chemischen Zustände noch genauer bestimmbar sind.

Weitere Vorteile ergeben sich aus der nachstehenden Beschreibung der Ausführungsbeispiele.

Zur selektiven Ausmessung von definierten, jedoch im Inneren
des zu untersuchenden Körpers frei wählbaren Punkten wird
erfindungsgemäß ein Verfahren der Lokalen Magnetischen
Resonanz (LMR) verwendet.

In einer ersten Ausgestaltung des erfindungsgemäßen Verfahrens können dabei zusätzlich zum homogenen Konstantmagnetfeld drei Gradientenfelder erzeugt werden, die nicht parallel, sondern vorzugsweise orthogonal verlaufen und sich
in einem gemeinsamen Schnittpunkt, nämlich dem zu untersuchenden Meßpunkt, schneiden. Werden nun alle drei Gradientenfelder zeitlich asynchron moduliert, so heben sich die Modulationen überall gegenseitig auf mit Ausnahme des Schnittpunktes, d.h. des zu untersuchenden Meßpunktes. In diesem Punkt, in dem die Modulationsamplitude folglich gleich Null ist, liegt demnach nur eine DC-Komponente vor, die leicht mit an sich bekannten Mitteln zu erfassen ist, so daß bei Verwendung dieses Verfahrens lediglich das LMR-Signal des Schnittpunktes der drei Ebenen erfaßt wird. Verfahren dieser Art sind in der Literatur beschrieben worden und werden dort in spezieller Ausgestaltung beispielsweise als "Sensitive Point"-Verfahren beschrieben, etwa im Aufsatz von Hinshaw in J.Appl. Phys. 47, Seite 3709 bis 3721 (1976).

Weiter kann der zu untersuchende selektive Meßpunkt dadurch festgelegt werden, daß dem homogenen Magnetfeld ein Gradientenfeld durch spezielle Zusatzspulen überlagert wird, bei dem lediglich ein eng umgrenzter Volumenbereich, nämlich der zu untersuchende Meßpunkt, über eine hohe Homogenität verfügt, während alle benachbarten Stellen, die den übrigen zu untersuchenden Körper umfassen, mit erheblicher Inhomogenität versehen werden, so daß an diesen inhomogenen Stellen keine nutzbaren Resonanzsignale erzeugt werden. Die selektive Homogenisierung des Magnetfeldes führt demnach zu einer selektiven Resonanzmessung. Auch dieses Verfahren ist in der Literatur beschrieben und wird in einer speziellen Ausgestaltung als "FONAR"-Verfahren bezeichnet, beispielsweise von Damadian in Physiol. Chem. Phys. 8, Seite 61 bis 65 (1976).

Schließlich kann in weiterer Ausgestaltung des erfindungsgemäßen Verfahrens ein LMR-Verfahren verwendet werden, wie dies in der älteren europäischen Patentanmeldung 83 102 169.6 der Anmelderin beschrieben ist.

Durch diesen Hinweis wird der gesamte Offenbarungsinhalt dieser älteren Patentanmeldung zum Offenbarungsgehalt der vorliegenden Anmeldung gemacht. Bei dem dort beschriebenen Verfahren wird dem homogenen Konstantmagnetfeld ein erstes, gleichgerichtetes Magnetfeld überlagert, dessen Stärke sich in Richtung einer ersten Raumkoordinate des Körpers ändert (erstes Gradientenfeld). Anschließend werden in dem Körper enthaltene, ausgewählte Kernspins, die sich in einer zur Richtung der ersten Raumkoordinate senkrecht stehenden, ausgewählen Körperebene befinden, welche durch die Larmorfrequenz der ausgewählten Kernspins in dem durch Überlagerung des homogenen Magnetfeldes und des ersten Gradientenfeldes resultierenden Magnetfeld definiert ist, selektiv angeregt, wonach ein weiteres Gradientenfeld angelegt wird, das wiederum zum homogenen Feld gleichgerichtet ist und sich in einer zur Richtung der ersten Raumkoordinate senkrechten Richtung ändert. Schließlich werden die Induktionssignale, welche von den dem homogenen Magnetfeld und dem weiteren Gradientenfeld ausgesetzten, angeregten Kernspins geliefert werden, zu Bildsignalen verarbeitet und bei angelegtem ersten Gradientenfeld wird zusätzlich die Gesamtheit der sich innerhalb des homogenen Magnetfeldes befindenden, ausgewählten Kernspins angeregt. Die selektive Anregung der in der ausgewählten Körperebene enthaltenen Kernspins erfolgt in der Weise, daß die in dieser Ebene enthaltenen, ausgewählten Kernspins derart in die Richtung des homogenen Magnetfeldes zurückgebracht werden, daß sie zu diesem Magnetfeld wahlweise entweder gleich oder entgegengesetzt gerichtet sind, wobei nach Anlegen des weiteren Gradientenfeldes eine erneute Anregung der in der ausgewählten Ebene enthaltenen, ausgewählten Kernspins erfolgt, so daß diese Kernspins das gewünschte Induktionssignal liefern. Dabei werden endlich die Messungen paarweise mit abwechselnd
gleich und entgegengesetzt zum homogenen Magnetfeld zurückgedrehten Kernspins ausgeführt und die erhaltenen Induktionssignale voneinander subtrahiert.

All diesen drei genannten LMR-Verfahren ist gemeinsam, daß
sie mit rein elektronischen Maßnahmen nacheinander die
Ausmessung selektiver Punkte mit großer Meßgeschwindigkeit
ermöglichen. Dabei wird der zu untersuchende Körper keinerlei Belastung ausgesetzt, da die verwendeten Konstant- und
Hochfrequenzmagnetfelder keine spürbaren physiologischen
Auswirkungen haben.

Erfindungsgemäß ist vorgesehen, an den eingestellten Meßpunkten einmal die jeweilige Temperatur des Meßpunktes oder
den dort herrschenden pH-Wert zu ermitteln.

Bei der hochauflösenden Kernresonanz treten bei untersuchten
Substanzen im Kernresonanzspektrum eine oder mehrere Linien
auf, die gegenüber der Resonanzlinie des freien Protons um
einen charakteristischen Betrag, die sog. chemische Verschiebung, versetzt sind. Diese, nur mit hochauflösenden
Kernresonanzmethoden zu ermittelnde Meßgröße läßt Rückschlüsse auf verschiedene chemische und/oder physikalische
Zustände zu. So ist es aus der Literatur bekannt, daß die
chemische Verschiebung einmal einen Aufschluß über die
Temperatur und zum anderen über den pH-Wert zuläßt. Dies ist
beispielsweise in Aufsätzen von van Geet, A.L.; Anal. Chem.
40, Seite 2227 bis 2229 (1968) für die Temperaturabhängigkeit und von Moon R.B., J. Biol. Chem. 248, Seite 7276
(1973) für den pH-Wert beschrieben.

Da die Magnetische-Kernresonanz mit einer Vielzahl von Kernarten eingesetzt werden kann, steht entsprechend eine noch größere Vielzahl von chemischen Substanzen zur Verfügung, die mit Hilfe der Kernresonanz untersucht werden kann. Erfindungsgemäß ist dabei einmal vorgesehen, im Körper ohnehin vorhandene Substanzen für die Messungen heranzuziehen, in weiterer Ausgestaltung der Erfindung ist jedoch auch vorgesehen, dem Körper gezielt Substanzen zuzuführen, beispielsweise zu injizieren, die besondere Kernresonanzsignale liefern, so daß verbesserte Messungen möglich sind. Selbstverständlich sind dabei nicht alle aus der Literatur bekannten Verbindungen verwendbar, die Kernresonanzsignale liefern, da diese Verbindungen zum Teil toxisch sind. Es versteht sich daher, daß für das erfindungsgemäße Verfahren nur nichttoxische Verbindungen verwendet werden oder andere Verbindungen in nichttoxischer Konzentration.

Für die Bestimmung der Temperatur eignen sich insbesondere die Kernarten $^1$H, $^2$H, $^{13}$C, $^{15}$N, $^{23}$Na, $^{31}$P, $^{59}$Co. Diese Kernarten finden sich - wie oben ausgeführt - entweder in körpereigenen Substanzen, insbesondere $^1$H in Histidin oder $^{31}$P in anorganischem oder organischem Phosphat in Form von ATP bzw. ADP. Für die Zuführung von Fremdstoffen in den zu untersuchenden Körper, die damit die Wirkung eines "NMR-Kontrastmittels" in Analogie zu den bei der Röntgentechnik verwendeten Kontrastmitteln, ausüben, lassen sich mit Vorteil die in Tabelle 1 aufgeführten Substanzen verwenden, die in den in Tabelle 2 angegebenen Literaturstellen erwähnt sind. Wie bereits betont, handelt es sich dabei lediglich um solche Substanzen, die von der Literatur als besonders geeignet für Kernresonanzmessungen bezeichnet werden, ohne Rücksicht darauf, ob sie für den menschlichen oder tierischen Körper schädlich sind oder nicht.

Für den Fall der Messung des pH-Wertes eignen sich erfindungsgemäß insbesondere die Kernarten $^{1}$H oder $^{31}$P. Dabei können diese Kernarten wieder in körpereigenen Substanzen, wie sie oben bereits erwähnt wurden, gemessen werden. Selbstverständlich ist auch eine entsprechende Zuführung von körperfremden Substanzen in der oben beschriebenen Weise möglich.

Wie bereits oben erwähnt, kann bei Verwendung der kernmagnetischen Resonanz auf eine Vielzahl von Kernarten zurückgegriffen werden. Es ist daher in Anlehnung an aus der Technik der Kernresonanzspektrometrie bekannte Verfahren auch möglich, mehrere unterschiedliche Kernarten gleichzeitig anzuregen und zu ermitteln, um die Variationsbreite der Aussagen der hochauflösenden lokalen Kernresonanzmessungen gemäß der vorliegenden Erfindung zu erhöhen.

Da die definierten Meßpunkte - wie ebenfalls oben erwähnt - durch rein elektronische Meßmittel eingestellt werden, ist es ohne Probleme möglich, nicht nur einzelne Punkte, sondern auch mehrere Punkte, vorzugsweise Flächen auszumessen, so daß komplette Flächenraster des gemessenen Parameters aufgezeichnet werden können. Damit können beispielsweise Temperaturprofile oder pH-Profile eines interessierenden Körperschnittes angegeben werden. Auf diese Weise kann die Temperaturverteilung in einem Körperquerschnitt, etwa durch mehrere Organe hindurch, angegeben werden.

Bei der hochauflösenden Kernresonanz ist es weiterhin bekannt, sog. Shim-Spulen vorzusehen, mit denen von Messung zu Messung eine extreme Homogenität am Probenort eingestellt wird, um besondes hochauflösende Messungen zu ermöglichen.

In weiterer Ausgestaltung der Erfindung ist daher schließlich noch vorgesehen, derartige zusätzliche Spulen einzusetzen, die so nach Art von Helmholtz-Spulen so gestaltet sind, daß sie eine Homogenisierung immer nur an einem bestimmten, vorgegebenen Ort bewirken. Durch diese lokal wirksamen Shim-Spulen wird dabei erreicht, daß jeweils nur der zu untersuchende Meßpunkt zusätzlich homogenisiert wird, um die hochauflösenden Messungen zu verbessern, beispielsweise die Genauigkeit der ermittelten chemischen Verschiebung, so daß die Präzision der ermittelten physikalischen und/oder chemischen Zustände ebenfalls erhöht wird.

In einem praktischen Beispiel einer Ausführungsform eines erfindungsgemäßen Verfahrens kann insgesamt demnach wie folgt vorgegangen werden:

In den zu untersuchenden Körper, in dem die Temperaturverteilung gemessen werden soll, wird die zur Messung vorgesehene Kernart, etwa $^1$H durch Injizieren von diversen Alkoholen in nicht toxischer Dosierung eingebracht. Dann wird der zu untersuchende Körper in das Konstantmagnetfeld eines NMR-Tomographen, etwa vom Typ CXP 100 t der Firma Bruker eingebracht. Zur Anregung der magnetischen Resonanz der vorgesehenen Kernart wird dabei z.B. ein 90°-Impuls benutzt. Nach Anregung der Kernresonanz werden die durch die Resonanzschwingungen induzierten Hochfrequenzsignale als Summensignal (Interferogramm) empfangen, aufgezeichnet und aus diesem mittels Fourier-Analyse (FFT) die einzelnen Resonanzschwingungen bzw. das Spektrum der erfaßten Substanz ermittelt. Dies erfolgt im einzelnen in einer Weise, wie sie aus der hochauflösenden FFT-NMR-Spektroskopie bekannt ist. Dabei kann beispielsweise als charakterisierender Spektralparameter die chemische Verschiebung einer oder mehrerer Linien absolut, in bezug auf eine Eichlösung der interessierenden Kernart oder im Hinblick auf mehrere verschiedene Kernarten ausgemessen werden. Die Temperatur wird dann als Funktion des Verschiebungswerts und ggf. weiterer Parameter (Feldstärke usf.) einem Kennfeld entnommen, das etwa in einen elektronischen Speicher (PROM) abgelegt ist.

Bei der vorstehend beispielhaft beschriebenen Messung hängt die Temperaturgenauigkeit in den einzelnen Volumenbereichen des Körpers abgesehen von der erwähnten Temperaturabhängigkeit der chemischen Verschiebung der Meßstubstanz auch von der Größe und Homogenität des verwendeten Konstantmagnetfeldes ab. Bei einem üblicherweise verwendeten und praktikablen Konstantmagnetfeld von etwa 1,9 T und ca. 20 cm Durchmesser des zu untersuchenden Körpers läßt sich etwa mit einer Genauigkeit von ± 0,2 K rechnen. Dies ist für die meisten medizinischen Untersuchungszwecke absolut ausreichend und ermöglicht in Anbetracht des insgesamt auftretenden Temperaturbereiches von lebenden Körpern auch die Erstellung von hinreichend aufgelösten und kontrastreichen Schnittbildern der Temperaturverteilung.

Entsprechendes gilt im praktischen Ausführungsbeispiel für die Messung des pH-Wertes.

Insgesamt versteht sich, daß die erfindungsgemäß verwendeten Verfahren, die angegebenen chemischen Untersuchungssubstanzen sowie schließlich die eingesetzten Geräte nur beispielhaft anzusehen sind und selbstverständlich auch andere Untersuchungsverfahren, die eine punktförmige Magnetische-Resonanz-Untersuchung gestatten, erfindungsgemäß verwendet werden können. Auch sind zahlreiche, der Literatur zu entnehmende Untersuchungsstoffe, sowohl körpereigene wie körperfremde, einsetzbar und die in der vorstehenden Beschreibung genannten sind in keiner Weise als Beschränkung auf einzelne Substanzen oder Substanzgruppen zu verstehen. Schließlich kann auch eine Vielzahl von an sich bekannten Untersuchungsgeräten zur Durchführung des erfindungsgemäßen Verfahrens verwendet werden, wobei hinsichtlich der verwendeten Kernart, der verwendeten Magnetfeldstärke bzw. der Meßfrequenz breite Variationsmöglichkeiten bestehen. Auch beschränkt sich das erfindungsgemäße Verfahren keineswegs auf die Kernresonanz, bei entsprechender Modifikation sind auch vergleichbare Elektronenresonanzmessungen möglich.

Patentansprüche

1. Verfahren zur nicht-invasiven Ermittlung chemischer
und/oder physikalischer Zustände innerhalb des gesamten, unversehrten, lebenden, tierischen oder menschlichen Körpers unter Verwendung Magnetischer Resonanz
mit einem homogenen Konstantmagnetfeld und einem
Hochfrequenzmagnetfeld, wobei einzelne Bereiche des
Körpers durch eine hochauflösende Magnetische-Resonanz-Messung ausgemessen werden und frei wählbare, an
beliebigen Orten innerhalb des lebenden Körpers liegende Meßpunkte durch Anwendung einer auf auswählbare
Volumenbereiche begrenzten magnetischen Resonanz (LMR,
Local Magnetic Resonance, Lokalisierte Magnetische
Resonanz) untersucht werden, dadurch gekennzeichnet,
daß aus einem Parameter des gemessenen Kernresonanzspektrums die Temperatur innerhalb der ausgewählten
Volumenbereiche ermittelt wird.

2. Verfahren zur nicht-invasiven Ermittlung chemischer
und/oder physikalischer Zustände innerhalb des gesamten, unversehrten, lebenden, tierischen oder menschlichen Körpers unter Verwendung Magnetischer Resonanz
mit einem homogenen Konstantmagnetfeld und einem Hochfrequenzmagnetfeld, wobei einzelne Bereiche des Körpers durch eine hochauflösende Magnetische-Resonanz-
Messung ausgemessen werden und frei wählbare, an
beliebigen Orten innerhalb des lebenden Körpers liegende Meßpunkte durch Anwendung einer auf auswählbare

2          **0095124**

Volumenbereiche begrenzten magnetischen Resonanz (LMR,
Local Magnetic Resonance, Lokalisierte Magnetische
Resonanz) untersucht werden, dadurch gekennzeichnet,
daß aus einem Parameter des gemessenen Kernresonanzspektrums der pH-Wert innerhalb der ausgewählen Volumenbereiche festgestellt wird.

3.     Verfahren nach Anspruch 1 oder 2, dadurch gekennzeichnet, daß zur Erzeugung der LMR ein Verfahren verwendet
wird, bei dem durch Festlegung dreier nicht paralleler
Gradientenfelder im Konstantmagnetfeld drei Bildebenen
der Magnetischen Resonanz erzeugt werden, die in dem
zu untersuchenden Meßpunkt einen gemeinsamen Schnittpunkt aufweisen, und ferner die drei Gradientenfelder
vorzugsweise asynchron moduliert werden, so daß lediglich der Schnittpunkt modulationsfrei und selektiv
auswertbar ist, wobei dieses Verfahren in einer speziellen Ausgestaltung als "Sensitive Point"-Verfahren
nach Hinshaw bekannt ist.

4.     Verfahren nach Anspruch 1 oder 2, dadurch gekennzeichnet, daß zur Erzeugung der LMR ein Verfahren verwendet
wird, bei dem Homogenisierungsmittel für das Konstantmagnetfeld vorgesehen sind, die einem, dem ausgewählten Volumenbereich bzw. dem Meßpunkt entsprechenden
Bereich ein Feldprofil hoher Homogenität und den übrigen, dem zu untersuchenden Körper entsprechenden
Bereichen ein Feldprofil hoher Inhomogenität verleihen, so daß lediglich der homogene Bereich auswertbare
Resonanzsignale liefert, wobei dieses Verfahren in
einer speziellen Ausgestaltung als "FONAR"-Verfahren
nach Damadian bekannt ist.

5.    Verfahren nach Anspruch 1 oder 2, dadurch gekennzeichnet, daß zur Erzeugung der LMR ein NMR-Tomographieverfahren verwendet wird, bei dem dem homogenen Konstantmagnetfeld ein erstes, gleichgerichtetes Magnetfeld
      überlagert wird, dessen Stärke sich in Richtung einer
      ersten Raumkoordinate des Körpers ändert (erstes
      Gradientenfeld), anschließend in dem Körper enthaltene, ausgewählte Kernspins, die sich in einer zur
      Richtung der ersten Raumkoordinate senkrecht stehenden, ausgewählten Körperebene befinden, welche durch
      die Larmorfrequenz der ausgewählten Kernspins in dem
      durch Überlagerung des homogenen Magnetfeldes und des
      ersten Gradientenfeldes resultierenden Magnetfeld
      definiert ist, selektiv angeregt werden, wonach ein
      weiteres Gradientenfeld angelegt wird, das wiederum
      zum homogenen Feld gleichgerichtet ist und sich in
      einer zur Richtung der ersten Raumkoordinate senkrechten Richtung ändert, die Induktionssignale, welche von
      den dem homogenen Magnetfeld und dem weiteren Gradientenfeld ausgesetzten, angeregten Kernspins geliefert
      werden, zu Bildsignalen verarbeitet werden, wobei bei
      angelegtem erstem Gradientenfeld zusätzlich die Gesamtheit der sich innerhalb des homogenen Magnetfeldes
      befindenden, ausgewählten Kernspins angeregt wird und
      die selektive Anregung der in der ausgewählten Körperebene enthaltenen Kernspins in der Weise erfolgt, daß
      die in dieser Ebene enthaltenen, ausgewählten Kernspins derart in die Richtung des homogenen Magnetfeldes zurückgebracht werden, daß sie zu diesem Magnetfeld wahlweise entweder gleich oder entgegengesetzt
      gerichtet sind, daß nach Anlegen des weiteren Gradientenfeldes eine erneute Anregung der in der ausgewählten Ebene enthaltenen, ausgewählten Kernspins erfolgt, so daß diese Kernspins das gewünschte Induktionssignal liefern und die Messungen paarweise mit abwechselnd gleich und entgegengesetzt zum homogenen Magnetfeld zurückgedrehten Kernspins ausgeführt und die erhaltenen Induktionssignale voneinander subtrahiert werden.

6. Verfahren nach einem der Ansprüche 1 und 3 bis 5, dadurch gekennzeichnet, daß z.B. die chemische Verschiebung einer oder mehrerer der folgenden Kernarten ermittelt wird: $^1$H, $^2$H, $^{13}$C, $^{15}$N, $^{23}$Na, $^{31}$P, $^{59}$Co.

7. Verfahren nach Anspruch 6, dadurch gekennzeichnet, daß die Messung an körpereigenen Substanzen, beispielsweise Hystidin oder organischem oder anorganischem Phosphat, etwa ATP bzw. ADP vorgenommen wird.

8. Verfahren nach Anspruch 6 oder 7, dadurch gekennzeichnet, daß einer der folgenden Stoffe in den Körper eingebracht, insbesondere injiziert wird: Ethylenglykol ($^1$H), Mg ATP (Adenosin-Triphosphat) ($^{31}$C), Lösung von Co (acac)$_3$ in CDCl$_3$ ($^{59}$Co).

9. Verfahren nach einem der Ansprüche 2 bis 5, dadurch gekennzeichnet, daß z.B. die chemische Verschiebung einer der folgenden Kernarten ermittelt wird: $^1$H oder $^{31}$P.

10. Verfahren nach Anspruch 9, dadurch gekennzeichnet, daß die Messung an körpereigenen Substanzen, beispielsweise Hystidin oder organischem oder anorganischem Phosphat, etwa ATP bzw. ADP vorgenommen wird.

11. Verfahren nach Anspruch 9 oder 10, dadurch gekennzeichnet, daß ein Stoff in den Körper eingebracht, insbesondere injiziert wird, der vorzugsweise eine der im Anspruch 9 genannten Kernarten enthält.

12. Verfahren nach einem der vorhergehenden Ansprüche, dadurch gekennzeichnet, daß die chemische Verschiebung von mindestens zwei unterschiedlichen Kernarten gleichzeitig ermittelt wird.

13. Verfahren nach einem der vorhergehenden Ansprüche, dadurch gekennzeichnet, daß ein Flächenraster des chemischen und/oder physikalischen Zustandes, beispielsweise des pH-Wertes bzw. der Temperatur entsprechend einer Bildebene im zu untersuchenden Körper erzeugt wird.

14. Verfahren nach einem der vorhergehenden Ansprüche, dadurch gekennzeichnet, daß zur Verbesserung der Feldhomongenität am Orte des Meßpunktes weitere Homogenisierungsmittel in Form von Shim-Spulen vorgesehen sind, mit denen eine selektive Homogenisierung am vorbestimmten Meßpunkt auf elektrischem Wege möglich ist.

Einige in der NMR gebräuliche Eichflüssigkeiten z. Temperaturbestimmung (oberhalb Z.T.)

| Kernart | "Temp.-Sonde" | Temperaturabhängigk. der Chem. Versch. (i. °C) (i. Hz) * | Messung von $\Delta\gamma$ zwischen | Empfindlichkeit $(\Delta\gamma \cdot b \cdot \Delta T = 1°C)$ | Ref. |
|---|---|---|---|---|---|
| $^1H$ | Ethylenglykol | $T = -1,278 \cdot \Delta\gamma + 193,9$ | OH ⟶ CH$_2$ | ~ 0,8Hz | 1), 2) |
| $^{13}C$ | Dijodomethan m. Cyclooktan | $1/T = (-1.1104 \cdot \Delta\gamma + 2200,27)10^{-5}$ | CH$_2$ I$_2$ ⟶ cycok | ~ 1Hz | 3) |
| $^{31}P$ | Mg ATP (Adenosin-Triphosphat) | $T = -2,5732 \cdot \Delta\gamma + 726,7$ | $\alpha P$ ⟶ $\beta P$ ($\alpha$-$\beta$ Phosphat) | ~ 0,4Hz | 4) |
| $^{31}P$ | Lösg. (C$_6$ H$_5$)$_3$P + (C$_6$ H$_5$)$_3$Po in Toluol-d$_8$ | $T = -0,955 \cdot \Delta\gamma + 937,244$ | (C$_6$ H$_5$)$_3$P ⟶ (C$_6$ H$_5$)$_3$Po | ~ 1Hz | 5) |
| $^{59}Co$ | Lösg. K$_3$Co (CN)$_6$ in D$_2$O oder Co (acac)$_3$ in CDCl$_3$ | $T = 0,03519 \cdot \Delta\gamma$ <br> $T = 0,01679 \cdot \Delta\gamma$ | $\Delta\gamma^{59}C$ ⟶ $\Delta\gamma 0°C$ | 29Hz <br> 60Hz | 6) |

* Bezogen auf Bo = 1,8790T ($\triangleq$ 80MHz   $^1H$-Resonanz)

0095124

1) Friebolin, Schilling u. Pohl:
   Organ. Magn. Res.    12, 10, S. 569 (1979)

2) Ammann, Meier u. Mehrbach:
   Journ. Magn. Res.    46, S. 319 (1982)

3) Vidrin u. Peterson:
   Anal. Chem.          48, 9, S. 1301 (1976)

4) Gupta u. Gupta:
   Journ. Magn. Res.    40, S. 587 (1980)

5) Dickert u. Hellmann:
   Anal. Chem.          52, S. 996 (1980)

6) Levy, Bailey u. Wright:
   Journ. Magn. Res.    37, S. 353 (1980)

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | FR-A-2 441 842 (R.V. DAMADIAN) * Seite 1, Zeile 4 - Seite 3, Zeile 8; Seite 22, Zeile 2 - Seite 23, Zeile 38; Seite 24, Zeile 16 - Seite 25, Zeile 29 * | 1 | G 01 N 24/08 A 61 B 5/05 |
| A | | 2-4,6, 9,13 | |
| | --- | | |
| Y | ANALYTICAL CHEMISTRY, Band 50, Nr. 2, Februar 1978, Seiten 298-303, American Chemical Society, Ohio, USA D.W. VIDRINE et al.: "Feedback excitation nuclear magnetic resonance spectrometry and its application to simultaneous temperature measurement" * Abschnitt "Results" * | 1 | |
| | --- | | RECHERCHIERTE SACHGEBIETE (Int. Cl. 3) |
| X | DE-A-2 854 774 (BATTELLE-INSTITUT e.V.) * Seite 14, Zeile 16 - Seite 15, Zeile 18; Seite 27, Zeile 24 - Seite 30, Zeile 18 * | 2,6-10 | G 01 N 24/00 A 61 B 5/05 |
| A | GB-A-2 057 142 (EMI LTD.) * Seite 1, Zeilen 3-68 * | 1,2,6, 9 | |
| | ---     -/- | | |

Der vorliegende Recherchenbericht wurde für alle Patentansprüche erstellt.

| Recherchenort | Abschlußdatum der Recherche | Prüfer |
|---|---|---|
| DEN HAAG | 18-08-1983 | HORAK G.I. |

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | GB-A-2 056 088 (EMI LTD.)<br><br>* Seite 1, Zeilen 41-66 * | 1,2,5, 9 | |
| A | DE-A-2 755 956 (NATIONAL RESEARCH DEVELOPMENT CORP.)<br>* Seite 1, Zeile 1 - Seite 3, Zeile 2 * | 1,2,5 | |
| A,D | JOURNAL OF APPLIED PHYSICS, Band 47, Nr. 8, August 1976, Seiten 3709-3721, New York, USA<br>W.S. HINSHAW: "Image formation by nuclear magnetic resonance: The sensitive-point method" * Abschnitt "Introduction" * | 3 | |
| A | IEEE TRANSACTIONS ON BIOMEDICAL ENGINEERING, Band BME-22, Nr. 1, Januar 1975, Seiten 12-18, IEEE, New York, USA<br>R.A. BROOKS et al.: "Nuclear magnetic relaxation in blood" * Seite 17 * | 1,2 | **RECHERCHIERTE SACHGEBIETE (Int. Cl. 3)** |

Der vorliegende Recherchenbericht wurde für alle Patentansprüche erstellt.

| Recherchenort | Abschlußdatum der Recherche | Prüfer |
|---|---|---|
| DEN HAAG | 18-08-1983 | HORAK G.I. |